# EUROPEAN PATENT APPLICATION

(11) **EP 3 508 852 A1**
(43) Date of publication of application: **10.07.2019**
(21) Application number: 17846691.8
(22) Date of filing: 31.08.2017
(51) Int. Cl.: G01N 33/543, G01N 21/64

(54) **ANALYTE CONCENTRATION MEASURING METHOD, PARTICLE CONTAINING AGGLUTINATED FLUORESCENT MATERIAL, AND INSPECTION DEVICE**

(30) Priority: 31.08.2016 JP 2016169030
(71) Applicant: Sekisui Chemical Co., Ltd., Osaka-shi, Osaka 530-8565 (JP); Sekisui Medical Co., Ltd., Tokyo 103-0027 (JP)
(72) Inventor: IWAMOTO Tadashi, Mishima-gun Osaka 618-0021 (JP); SUGIMOTO Satoru, Mishima-gun Osaka 618-0021 (JP); WAKIYA Takeshi, Mishima-gun Osaka 618-0021 (JP); KITAHARA Shinichiro, Tokyo 103-0027 (JP); YAJI Maasa, Tokyo 103-0027 (JP)
(74) Representative: Hoffmann Eitle
(86) International application number: PCT/JP2017/031500
(87) International publication number: WO 2018/043688

(57) **Abstract**

This analyte concentration measuring method including:
preparing a mixed solution by mixing a sample solution containing an analyte, with a solution containing aggregation-induced emission fluorescent material-containing particles that have a binding partner which binds with the analyte and that agglutinate and fluoresce when the analyte binds to the binding partner;
measuring the fluorescence intensity generated from the aggregation-induced emission fluorescent material-containing particles in the mixed solution; and
comparing a fluorescence intensity calibration curve for analyte concentration with the fluorescence intensity, and associating the fluorescence intensity with the analyte concentration in the mixed solution. Employing agglutinating-luminescent-material-containing particles enables measurements to be carried out with a satisfactory detection sensitivity while suppressing background fluorescence.

## Description

### TECHNICAL FIELD

The present invention relates to an analyte concentration measuring method, aggregation-induced emission fluorescent material-containing particles, and an inspection device.

### BACKGROUND ART

A method of measuring an analyte in a sample by detecting fluorescence (fluorescence method) enables convenient and highly sensitive measurement. Since this method may also be automated using an analyzer, such as an immuno-plate reader, etc., it has been used in various fields including a clinical test. The fluorescence method is very excellent from viewpoints of high efficiency, convenience and the like.

However, the method of measuring an analyte in a sample by detecting fluorescence may generate so-called background fluorescence, which is not derived from the analyte. Background fluorescence may be generated from autofluorescence of endogenous substances other than an analyte in a sample, from a fluorescent dye which is non-specifically attached to proteins or the like in a sample, or from a container (such as a plate) into which an analyte is introduced. Since any of the above cases may affect sensitivity and specificity, it is a common problem of the methods of measuring an analyte in a sample by detecting fluorescence. Accordingly, there has been a demand for a measurement method which is free from the influence of background fluorescence.

Patent Literatures 1 and 2 disclose an antibody which recognizes an analyte-dye complex of an analyte having a dye which is not substantially fluorescent, as an antigen. However, this antibody corresponds only to specific antigens, and in some cases, background fluorescence may not be reduced due to the influence of a plurality of proteins contained in the sample.

### CITATION LIST

### PATENT LITERATURE

Patent Literature 1: Japanese Patent Laid-Open No.1997-5324
Patent Literature 2: Japanese Patent Laid-Open No.2007-171213

### NON-PATENT LITERATURE

Non-Patent Literature 1: Journal of Synthetic Organic Chemistry: Vol. 71, No. 9, p961 (2013)

### SUMMARY OF INVENTION

### TECHNICAL PROBLEM

An object of the present invention is to provide an immunological measurement method that enables measurement with a satisfactory detection sensitivity while suppressing background fluorescence using aggregation-induced emission fluorescent material-containing particles.

### SOLUTION TO PROBLEM

The present invention includes the following descriptions:
(1) An analyte concentration measuring method including: preparing a mixed solution by mixing a sample solution containing an analyte with a solution containing aggregation-induced emission fluorescent material-containing particles that have a binding partner which binds with the analyte and that agglutinate and fluoresce when the analyte binds to the binding partner; measuring fluorescence intensity generated from the aggregation-induced emission fluorescent material-containing particles in the mixed solution; and comparing a fluorescence intensity calibration curve for an analyte concentration with the fluorescence intensity, and associating the fluorescence intensity with the analyte concentration in the mixed solution.
(2) The analyte concentration measuring method of (1), wherein in the step of measuring the fluorescence intensity, at least one of a step of measuring a variation in absorbance of the mixed solution from an absorbance difference between a first time point and a second time point and a step of measuring a scattered light intensity difference of the mixed solution from a scattered light intensity difference between a third time point and a fourth time point.
(3) The analyte concentration measuring method of (1) or (2), wherein in the step of associating the analyte concentration, the fluorescence intensity is associated with the analyte concentration using the variation of the absorbance and/or the variation of the scattered light intensity and a calibration curve based on the variation of the scattered light intensity and/or a calibration curve based on the variation of the absorbance.
(4) An aggregation-induced emission fluorescent material-containing particle including a core particle and an aggregation-induced emission fluorescent material provided on the core particle, wherein the aggregation-induced emission fluorescent material has a binding partner which binds with an analyte, and agglutinates and fluoresces when the analyte binds to the binding partner.
(5) The aggregation-induced emission fluorescent material-containing particle of (4), wherein the aggregation-induced emission fluorescent material has an agglutinating fluorescent site localized on an insoluble carrier.
(6) The aggregation-induced emission fluorescent material-containing particle of (5), wherein the aggregation-induced emission fluorescent material is provided as a graft chain on the surface of the insoluble carrier.
(7) The aggregation-induced emission fluorescent material-containing particle of any one of (4) to (6), wherein the aggregation-induced emission fluorescent material further includes a hydrophilic group.
(8) An inspection device including an insoluble carrier and a detection portion which is provided on the insoluble carrier, the detection portion including an aggregation-induced emission fluorescent material which has a binding partner which binds to an analyte and agglutinates and fluoresces when the analyte binds to the binding partner.
(9) The inspection device of (8), wherein the insoluble carrier is an insoluble membrane carrier.

### ADVANTAGEOUS EFFECTS OF INVENTION

The present invention may provide an immunological measurement method that enables measurement with a satisfactory detection sensitivity while suppressing background fluorescence by using aggregation-induced emission fluorescent material-containing particles.

### BRIEF DESCRIPTION OF DRAWINGS

FIGS. 1(A) and 1(B) are conceptual diagrams of an analyte concentration measuring method using aggregation-induced emission fluorescent material-containing particles;
FIG. 2(A) is a perspective view of a test strip, and FIGS. 2(B) and 2(C) show a usage state;
FIG. 3(A) is a perspective view of an inspection device including an aggregation-induced emission fluorescent material, FIG. 3 (B) is a cross-sectional view thereof, and FIG. 3 (C) is a conceptual diagram showing a usage state; and
FIG. 4(A) is a perspective view of a conventional immunochromatographic test strip, and FIGS. 4(B) and 4(C) show a usage state.

### DESCRIPTION OF EMBODIMENTS

Hereinafter, the present invention will be described with reference to embodiments, but the present invention is not limited to the following embodiments.

Conventional organic fluorescent dyes have a great problem that when they are used in a solution or in a solid state, dye molecules agglutinate with each other, and thus their functions, such as light emitting efficiency, a coloring property, photosensitivity, and a photosensitizing property, etc., remarkably deteriorate to restrict intrinsic properties of the dyes. However, several studies have recently reported on molecules which agglutinates to significantly improve the fluorescence quantum yield (e.g., see Non-Patent Literature 1). This phenomenon is also called aggregation induced emission (AIE), and its principle is thought to be attributable to restriction of the intermolecular structural change by aggregation. The advent of AIE is expected to overcome problems so far and to provide new applications of organic fluorescent dyes in medical and industrial fields, etc.

Currently, many measurement reagents for a microparticle enhanced light scattering agglutination assay using a carrier particles carrying a binding partner for the analyte are practically used in clinical diagnostics. However, there is a problem in ON-OFF control of binding of a binding partner to an analyte, and thus a system that enables easier ON-OFF control has been required. The present inventors have created to perform ON-OFF control of binding of a binding partner to an analyte by using AIE.

### [Analyte Concentration Measuring Method]

A measuring method according to an embodiment includes (a) a step of preparing a mixed solution by mixing a sample solution containing an analyte with a solution containing aggregation-induced emission fluorescent material-containing particles that have a binding partner which binds with the analyte and that agglutinate and fluoresce when the analyte binds to the binding partner; (b) a step of measuring fluorescence intensity generated from the aggregation-induced emission fluorescent material-containing particles in the mixed solution; and (c) a step of comparing a fluorescence intensity calibration curve for an analyte concentration with the fluorescence intensity, and associating the fluorescence intensity with the analyte concentration in the mixed solution. According to the present embodiment, based on agglutinating fluorescence characteristics of the aggregation-induced emission fluorescent material-containing particles, it is possible to measure the analyte concentration with easy ON-OFF control of the binding between the analyte and the binding partner. According to this measuring method, the presence or absence of the analyte and the analyte concentration may be accurately measured, even when the analyte concentration is low, by using aggregation-induced emission fluorescent material-containing particles with high sensitivity described below. Further, the analyte concentration may be measured over a wide range using an existing measuring device by using a measuring reagent consisting of a first reagent solution (R1) and a second reagent solution (R2) described below in an appropriate combination.

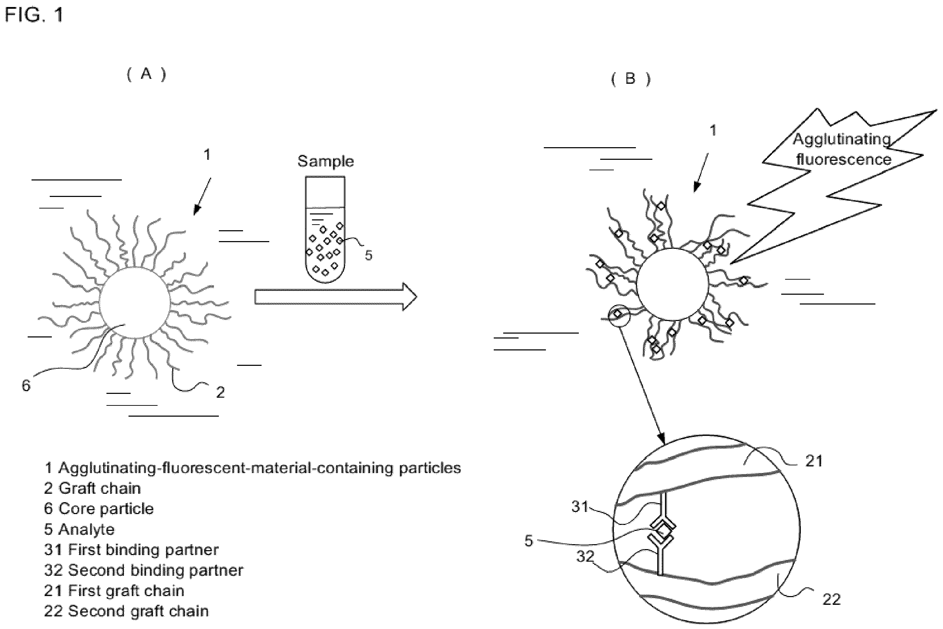

A mechanism of an immunoagglutination fluorescence assay will be briefly described below by taking as an example the case of using an aggregation-induced emission fluorescent material-containing particle 1 which is provided with graft chains 2 consisting of an aggregation-induced emission fluorescent material on the surface of a core particle 6, as shown in FIGS. 1(A) and 1(B). As shown in FIG. 1(A), in the solution containing aggregation-induced emission fluorescent material-containing particles 1, the graft chains 2 provided on the surface of the aggregation-induced emission fluorescent material-containing particles 1 vibrate in the solvent due to influence of a hydrophilic group, for example, a hydroxyl group in the graft chain 2. When this solution is mixed with a sample solution (specimen) containing an analyte 5, the analyte 5 binds to first and second binding partners 31, 32 of neighboring first and second graft chains 21, 22, resulting in occurrence of agglutination between the graft chains 21, 22, as in a partially enlarged view of FIG. 1(B). The agglutination of the graft chains 21, 22 is considered to generate fluorescence due to a decrease in the degree of rotational freedom of the graft chains (or a predetermined group in the graft chain). The present invention has been accomplished based on the above finding. Hereinafter, each step and the like will be described in detail.

In the step (b) of measuring fluorescence intensity, at least one of a step of measuring a variation in absorbance of the mixed solution from an absorbance difference between a first time point and a second time point and a step of measuring a scattered light intensity difference of the mixed solution from a scattered light intensity difference between a third time point and a fourth time point is preferably carried out.

In the step (c) of associating the analyte concentration, the fluorescence intensity is preferably associated with the analyte concentration using the variation of the absorbance and/or the variation of the scattered light intensity and a calibration curve based on the variation of the scattered light intensity and/or a calibration curve based on the variation of the absorbance.

According to the present embodiment, owing to these steps, it is possible to obtain a calibration curve substantially ranging from a low concentration to a high concentration, and it is possible to perform measurement with high sensitivity over a wide dynamic range.

Here, it is preferable that the first, second, third, and fourth time points are respectively selected from the start of the preparation of the mixed solution to 1000 seconds. When the time points are within 1000 seconds from the start of the preparation of the mixed solution, it is possible to satisfy both desired sensitivity and desired dynamic range while securing the degree of freedom of the design of the measuring reagent.

The measurement of the variation of the scattered light intensity and the variation of the absorbance are preferably performed using a common wavelength. Further, the measurement of the variation of the scattered light intensity and the variation of the absorbance are preferably performed within a wavelength range of 550 nm to 900 nm.

Hereinafter, the measuring method according to embodiments will be described in detail with explanation on the analyte used in the present embodiment.

Further, as used herein, the term "single measurement" refers to a series of reactions and measurements performed in a single reaction vessel. Taking measurement in an automated analyzer as an example, single measurement means that a first reagent solution is mixed with a sample, and subsequently, a second reagent solution (a solution containing insoluble carrier particles carrying a binding partner for the analyte) is added and mixed, and measurement of a variation of scattered light intensity and measurement of a variation of absorbance are carried out in a single reaction vessel.

Further, as used herein, the term "sample solution containing an analyte" includes a sample solution which is mixed and diluted with a first reagent solution (a buffer solution) as described above.

Further, as used herein, the term "scattered light intensity" may be also written as "degree of scattered light", but they have the same meaning.

### (Aggregation-induced emission fluorescent material-Containing Particle)

The aggregation-induced emission fluorescent material-containing particle will be described below.

### (Insoluble Carrier)

In the measuring method of the present invention, a material used as the insoluble carrier is not particularly limited as long as it is a material that is applicable as a component of the measuring reagent. Specifically, latex, metal colloid, silica, carbon or the like may be mentioned. An average particle diameter of the insoluble carrier particles may be appropriately selected from 0.05 µm to 1 µm. However, in the measuring reagent of the present invention, a particle size which is smaller than the wavelength of the light irradiated during the measurement of the scattered light intensity with a difference of 250 nm to 450 nm, specifically, with a difference of 300 nm to 450 nm is preferred. For example, when the irradiated wavelength is 700 nm, the average particle diameter is 250 nm to 400 nm. The average particle diameter of the insoluble carrier particles may be confirmed by a method generally used in a particle size distribution analyzer, a transmission electron microscopy or the like.

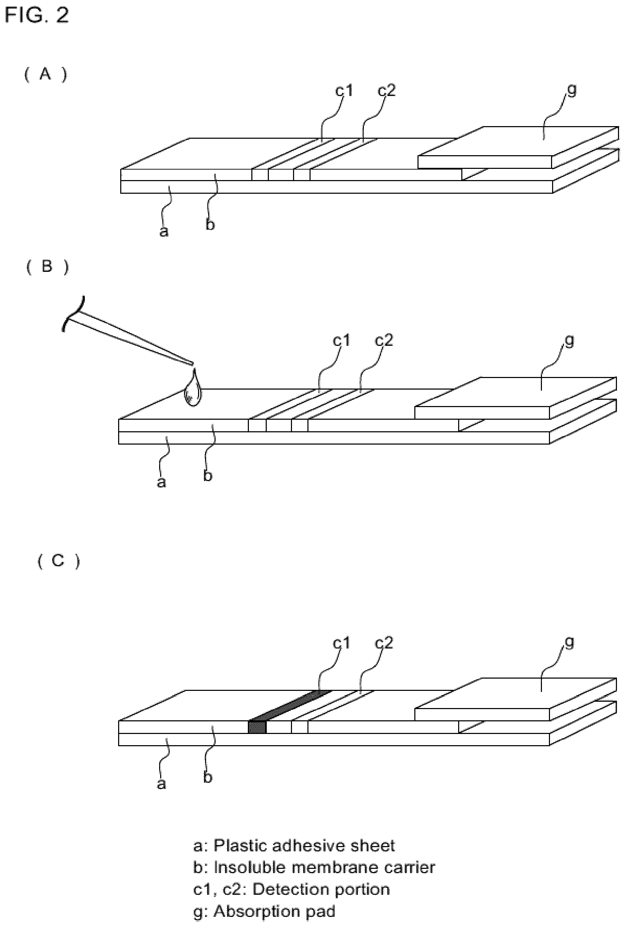

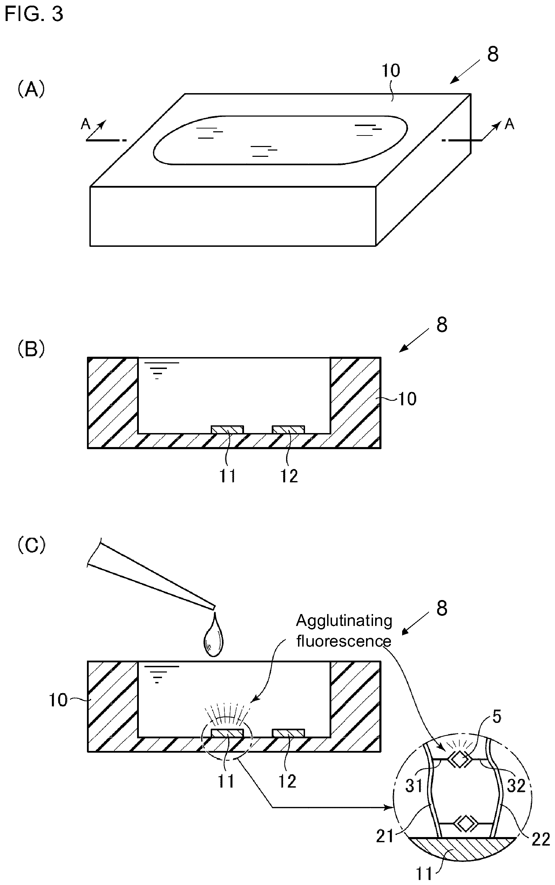

In addition to those mentioned above, an insoluble membrane carrier and a plastic material may also be used as the insoluble carrier, as explained in FIGS. 2 and 3 below. The plastic material is not particularly limited, but it may be exemplified by polyethylene, polypropylene, polycarbonate, and the like.

### (Sample)

The measuring method of the present invention is applicable to measurement of various types of biological samples including, but not particularly limited to, body fluids such as blood, serum, plasma, urine, or the like.

### (Analyte)

The analyte of the measuring method is not particularly limited as long as it is a molecule which may be theoretically measured by the measuring method, such as proteins, peptides, amino acids, lipids, sugars, nucleic acids, haptens, etc. Examples thereof may include CRP (C reactive protein), Lp (a) (lipoprotein (a)), MMP 3 (matrix metalloproteinase 3), anti-CCP (cyclic citrullinated peptide) antibody, anti-phospholipid antibody, an anti-syphilis antigen antibody, RPR, type IV collagen, PSA, AFP, CEA, BNP (brain natriuretic peptide), NT-proBNP, insulin, microalbumin, cystatin C, RF (rheumatoid factor), CA-RF, KL-6, PIVKA-II, FDP, D-dimer, SF (soluble fibrin), TAT (thrombin-antithrombin III complex), PIC, PAI, factor XIII, pepsinogen I, pepsinogen II, phenytoin, phenobarbital, carbamazepine, valproic acid, theophylline and the like.

### (Binding Partner)

The binding partner provided in the measuring method of the present invention may include proteins, peptides, amino acids, lipids, sugars, nucleic acids, haptens, and the like as a material that binds to an analyte, but antibodies and antigens are generally used, in view of their specificity and affinity. There are no particular limitation in its molecular weight and origin, either naturally occurring or being synthesized.

### (Measuring Reagent)

A composition of the measuring reagent which is provided in the measuring method of the present invention is not particularly limited, but considering use of the measuring reagent in an automated analyzer generally used in the field of clinical tests, the measuring reagent is generally composed of two solutions of a first reagent solution (R1) containing a buffer solution and a second reagent solution (R2) containing carrier particles which carry binding partners for the analyte.

### (Components of Measuring Reagent)

In addition to the insoluble carrier carrying binding partners which are a main component for reaction, components of the measuring reagent using the insoluble carrier particles of the present invention may include a component for buffering the ionic strength or osmotic pressure of the sample, for example, acetic acid, citric acid, phosphoric acid, Tris, glycine, boric acid, carbonic acid, Good's buffer, and sodium salts, potassium salts, and calcium salts thereof, etc. The reagent may also include, as a component for enhancing agglutination, polymers such as polyethylene glycol, polyvinyl pyrrolidone, phospholipid polymer, etc. The reagent may also include, as a component for controlling agglutination, one or more of generally used components, such as macromolecular substances, proteins, amino acids, sugars, metal salts, surfactants, reducing substances, chaotropic substances, etc. The reagent may also include an antifoaming substance.

### (Analyzer)

Use of a quick and simple automated analyzer that requires a total reaction time of 10 minutes or less for the measurement is suitable for the measuring method of the present invention. An automated analyzer capable of measuring the scattered light intensity and the absorbance substantially at the same time is preferred, as disclosed in JP-A-2013-64705.

### (Scattering Angle)

A scattering angle used in the measurement of the scattered light intensity of the present invention is not particularly limited, but the scattering angle is preferably 15 to 35 degrees, and more preferably 20 to 30 degrees. The scattering angle within the above range prevents excessive influences of transmitted light on a light receiver for detection of the scattered light, and is advantageous for its ability to receive the scattered light.

### (Measurement of Scattered Light Intensity)

Although a light source and a wavelength of irradiated light for measuring the scattered light intensity in the present invention are not particularly limited, a visible light region, specifically, 650 nm to 750 nm is suitable for the measuring method. The time intervals of two time points at which the variation in the scattered light intensity is measured are not particularly limited. In general, higher sensitivity is provided when the time intervals are longer.

The automated analyzer described above may individually measure the variation in the scattered light intensity and the variation in absorbance at any two time points selected between 0 and at most 1000 seconds immediately after mixing the sample solution containing the analyte with a solution containing insoluble carrier particles which carry binding partners for the analyte. When the individual variations in the scattered light intensity and the absorbance are measured at two time points between 0 and 300 seconds immediately after the mixing, the total measurement time of single measurement (for a single sample) with the first and second reagent solutions may be reduced to 10 minutes or less, and it is possible to provide the benefit of the highest sample analysis speed of various commercially available automated analyzers.

### (Measurement of Absorbance)

Although a wavelength for absorbance measurement in the present invention is not particularly limited, the same or different wavelength within a range of ±25% of a wavelength at which the variation in scattered light intensity is suitable. A range of 550 nm to 900 nm is preferred and a range of 570 nm to 800 nm is more preferred. The wavelength for absorbance measurement in the present invention may be either at a single wavelength or at two wavelengths of a combination of a main wavelength on the shorter wavelength side and a sub wavelength on the longer wavelength side than the wavelength used in the measurement of the scattered light intensity, within the aforementioned ranges. For example, when the measurement wavelength of the scattered light intensity is set at 700 nm, the main and sub wavelengths for the measurement of the absorbance may be set in the ranges of from 550 nm to 699 nm and from 701 nm to 900 nm, respectively.

The time intervals of two time points at which the variation in the absorbance is measured are not particularly limited, and are suitably shorter than, and preferably, 1/2 or less of those at which the scattered light intensity is measured. Further, the time intervals are preferably 1/3 or less of those at which the scattered light intensity is measured. For example, when the time intervals at which the variation in the scattered light intensity is set at 300 seconds, the time intervals at which the variation in the absorbance is measured may be preferably 150 seconds or less, and more preferably 100 seconds or less. The measurement is preferably started immediately after mixing the sample solution containing the analyte with the solution containing insoluble carrier particles carrying binding partners for the analyte.

### (Variations)

The variations in the light quantity (scattered light intensity and absorbance) used in the present invention may be measured by any calculation method without limitation, as long as it is applicable to particle enhanced agglutination immunoassay, including calculation of a difference, a ratio, and a corresponding value per unit time for the two time points.

### (Step of Associating with Presence Amount of Analyte)

In the measuring method according to embodiments, a sample containing a known concentration of analyte is used to analyze the scattered light intensity and the absorbance to plot individual calibration curves. At a low concentration of the analyte, the concentration is determined based on the calibration curve plotted based on the measurement of the scattered light intensity, in which high sensitivity is achieved, and at a high concentration of the analyte, the concentration is determined based on the calibration curve plotted based on the measurement of the absorbance, in which a wide dynamic range is achieved. The determination based on the absorbance provides a wide dynamic range, and allows to plot a calibration curve which covers a wider concentration range.

### (Sensitivity and Dynamic Range)

The "sensitivity" refers to a minimum measurable amount of an analyte. In general, larger variations in light quantity associated with the amount of analyte means higher sensitivity. In the measuring method according to embodiments, the high sensitivity is indicated by high accuracy and reproducibility of the measurements for the amount of the analyte at a low concentration.

The dynamic range refers to a range of a maximum measurable amount of an analyte. In the measuring method according to embodiments, the dynamic range represents a range within which variations in light quantity proportional to the analyte concentration may be detected.

The sensitivity and dynamic range of a particle enhanced agglutination immunoassay are dependent on insoluble carrier particles which are contained in the measuring reagent and carry binding partners. These two characteristics are in a trade-off relation, as described above.

### [Aggregation-induced emission fluorescent material-Containing Particles]

The aggregation-induced emission fluorescent material-containing particles which may be used in the above-described measuring method according to embodiments have a core particle and an aggregation-induced emission fluorescent material provided on the core particle, wherein the aggregation-induced emission fluorescent material has a binding partner which binds with an analyte, and agglutinates and fluoresces when the analyte binds to the binding partner. The aggregation-induced emission fluorescent material preferably has an agglutinating fluorescent site localized on the surface of the particle. The aggregation-induced emission fluorescent material is preferably provided as a graft chain on the surface of the core particle. Further, the aggregation-induced emission fluorescent material preferably has a hydrophilic group.

### (Core particle)

As the core particles, organic polymer microparticles may be used. The organic polymer microparticles may be particles composed of a copolymer obtained by copolymerizing (1) one or more polymerizable monomers selected from the group consisting of a polymerizable monomer having a phenyl group, a polymerizable monomer having a methacryloyl group, and a polymerizable monomer having an acryloyl group, and (2) a polymerizable monomer containing a graft polymerization initiator group. The organic polymer microparticles are not particularly limited, and particles which have been conventionally used in the immunoassay field may be used.

A polymerizable monomer having a phenyl group may include, for example, polymerizable unsaturated aromatic monomers such as styrene, chlorostyrene, α-methylstyrene, vinyltoluene or the like. A crosslinkable monomer such as divinylbenzene or the like may also be included in an appropriate amount. A polymerizable monomer having a methacryloyl group or an acrylic group may include, for example, polymerizable unsaturated carboxylic acid esters such as methyl (meth)acrylate, ethyl (meth)acrylate, ethyl n-propyl (meth)acrylate, 2-hydroxyethyl (meth)acrylate, glycidyl (meth)acrylate or the like, polymerizable unsaturated carboxylic acids such as (meth)acrylic acid, itaconic acid, maleic acid, fumaric acid or the like, or salts thereof, for example, sodium (meth)acrylate or potassium (meth)acrylate, and polymerizable unsaturated carboxylic acid amides such as (meth)acrylamide, N-methylol (meth)acrylamide, N,N-dimethyl (meth)acrylate or the like. A crosslinkable monomer such as ethylene glycol (meth)acrylate, propylene glycol (meth)acrylate, methylene bis (meth) acrylamide and the like may also be included in an appropriate amount. These monomers may be used alone or in combination of two or more thereof.

Among them, a copolymer composed of styrene and 2-chloropropionyloxyethyl methacrylate (hereafter, also referred to as CPEM) and a copolymer composed of styrene, methyl methacrylate and CPEM are particularly preferred.

Further, the amount of the monomer of (2) is an important factor that determines its density since it becomes a starting point of the graft chain described below. If the amount is too small, the starting point is small, the density of the graft chain decreases, leading to color deterioration. If the amount is too large, problems such as deterioration in monodispersibility and dispersion stability of the particles may be generated. Therefore, the amount is preferably 0.1 mol% to 20 mol% with respect to the total amount of (1), but it may be arbitrarily selected according to characteristics of an immunochromatographic reagent.

As a method of polymerizing the copolymer, a known polymerization method may be used, such as a dispersion polymerization method, a suspension polymerization method, an emulsion polymerization method, and a soap-free emulsion polymerization. The soap-free emulsion method is preferred.

In the soap-free polymerization, a polymerization initiator is required, in addition to the monomer constituting one organic polymer microparticle of the present invention. A water-soluble monomer, other additives and the like may also be appropriately added.

As the polymerization initiator, a conventionally known polymerization initiator may be used. For example, an aqueous solution such as potassium persulfate, sodium persulfate, ammonium persulfate or the like may be used as a water-soluble anionic initiator. An aqueous solution such as 2,2'-azobis (2-amidinopropane)dihydrochloride (hereinafter, referred to as "V-50"), 2,2'-azobis [2-(2-imidazolin-2-yl)propane] dihydrochloride, 2,2'-azobis (1-imino-1-pyrrolidino-2-methylpropane)dihydrochloride or the like may be used as a water-soluble cationic initiator. Among them, the water-soluble cationic initiator is preferred, and V-50 is more preferred.

As a trace amount of water-soluble monomer, any of cationic, anionic, and nonionic monomers may be used. The cationic monomer may include N-n-butyl-N-(2-methacryloyloxy)ethyl-N,N-dimethylammonium bromide (hereinafter, referred to as "C4-DMAEMA"), N-(2-methacryloyloxy)ethyl-N,N,N-trimethylammonium chloride and the like. The anionic monomer may include acrylic acid, methacrylic acid, styrene sulfonic acid and the like. The nonionic monomer may include acrylamide, polyethylene glycol monomethoxy methacrylate and the like. The cationic monomer is preferred, and C4-DMAEMA is more preferred.

Other additives may include alcohols such as methanol, ethanol and the like and may be used in appropriate amounts.

A preferred range of the particle size of the polymer particle is 50 nm to 300 nm, and a more preferred range of the particle size is 200 nm to 300 nm.

### (Aggregation-induced emission fluorescent material)

The aggregation-induced emission fluorescent material (AIE) constituting fluorescent particles for a diagnostic agent is not particularly limited, but examples thereof may include ketoimine boron complex derivatives, diimine boron complex derivatives, tetraphenylethylene derivatives, aminomaleimide derivatives, aminobenzopyroxanthene derivatives, triphenylamine derivatives, hexaphenylbenzene derivatives, hexaphenylsilole derivatives and the like. Among the above-mentioned derivatives, the tetraphenylethylene derivatives are preferred, because they are easy to synthesize and are also commercially available.

Examples of the tetraphenylethylene derivatives may include ethylene derivatives substituted with four or more phenyl groups or phenyl derivatives. Specifically, an ethylene derivative represented by the following formula (1) may be mentioned: 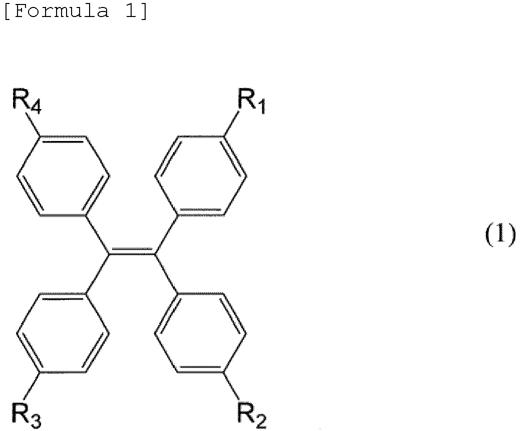 (wherein R₁ represents any one of a hydrogen atom, a bromine atom, and a hydroxyl group, and R₂, R₃ and R₄ represent a hydrogen atom or a hydroxyl group, respectively.)

More specifically, tetraphenylethylene, 1-(4-bromophenyl)-1,2,2-triphenylethylene, and tetrakis(4-hydroxyphenyl)ethylene may be mentioned. Further, the tetraphenylethylene derivative of Formula 1 is preferably graft-polymerized onto the surface of the core particle composed of a synthetic polymer by elimination of R₃.

Examples of the hexaphenylbenzene derivative may include benzene derivatives substituted with four or more phenyl groups or phenyl derivatives. Specifically, hexaphenylsilole or hexaphenylbenzene may be mentioned.

Examples of the triphenylamine derivative may include 4-(di-p-triamino)benzaldehyde.

A number average molecular weight of the aggregation-induced emission fluorescent material is preferably 10,000 or less. If the number average molecular weight exceeds the upper limit, the aggregation-induced emission fluorescent material is hard to dissolve, and thus it may not be processed into a particle shape, or its content tends to decrease.

Each of the above-mentioned derivatives is preferably graft-polymerized onto the surface of the core particle composed of a synthetic polymer by elimination of a part of the groups. In addition to direct graft polymerization of the above-mentioned derivative onto the surface of the core particle, the above derivative may be graft-polymerized to the core particle in the form of being incorporated into the main chain or side chain of the polymer.

A method of introducing the binding partner binding with the analyte into the aggregation-induced emission fluorescent material is not particularly limited, and a method of providing the binding partner on the surface of the aggregation-induced emission fluorescent material via a binder may be exemplified. Specifically, a method of coating the surface of the aggregation-induced emission fluorescent material with a material having a primary amino group, and then introducing the binding partner thereto may be used. By such a method, the binding partner is formed by binding the primary amino group to the surface of the aggregation-induced emission fluorescent material particles via a sulfur atom. Examples of the material having the primary amino group may include thiols having primary amino groups, such as 2-aminoethanethiol, 3-aminopropanethiol, 4-aminobutanethiol and the like. Among them, 2-aminoethanethiol is preferred.

Particles having an average particle diameter of 100 nm to 2000 nm, preferably 200 nm to 1000 nm, and more preferably 300 nm to 800 nm may be used as the aggregation-induced emission fluorescent material-containing particles. A CV value (coefficient of variation of the particle size) is preferably 10% or less. The CV value is calculated from "standard deviation of particle size distribution ÷ average particle diameter × 100". When the average particle diameter of the composite particles is less than 100 nm, the visibility deteriorates, and when it exceeds 2000 nm, the possibility of causing clogging in the membrane is increased. As used herein, the average particle diameter means an average of values obtained by analyzing 100 or more of particle images in any one field of view which is obtained by a scanning electron microscope.

The average particle diameter of the aggregation-induced emission fluorescent material-containing particles may be controlled, for example, either at the time of forming the organic polymer microparticles or at the time of forming the graft chains. Considering the performance of the measuring method and ease of preparation, an optimum particle size may be preferably controlled in combination of the time of forming the organic polymer microparticles and the time of forming the graft chains. For example, the total particle diameter may be set to 700 nm by setting the organic polymer microparticles of 200 nm with the graft chains of a length of 250 nm, or the total particle diameter may be set to 700 nm by setting the organic polymer microparticles of 500 nm with the graft chains of a length of 100 nm.

As a method of imparting the graft chains to the organic polymer microparticles, a conventional polymerization method known as a controlled/living radical polymerization may be used, and examples thereof may include atom transfer radical polymerization (ATRP), nitroxide-mediated polymerization (NMP), reversible addition fragmentation chain transfer (RAFT) polymerization and the like, but ATRP is preferred. These may be performed using a method described in, for example, K. Matyjaszweski, J. Xia, Chem. Rev., 101 (2001), pp. 2921-2990, M. Kamigaito, T. Ando, M. Sawamoto, Chem. Rev., 101 (2001), pp. 3689-3745.

A transition metal complex used in ATRP may reversibly generate carbon radicals by oxidation-reduction reaction of one electron. A central metal may include ruthenium, copper, iron, nickel, palladium, rhodium, cobalt, rhenium, manganese, molybdenum and the like. A ligand may include multidentate amine, pyridine, phosphine, cyclopentadiene and the like, and combination of the ligand with the central metal properly controls the activity of the transition metal catalyst. When a high valent transition metal is used, it is also possible to produce a low valent transition metal using ascorbic acid, a sugar, divalent tin or the like.

A chain length of the graft chain is not particularly limited as long as the overall particle diameter is 100 nm to 700 nm. When used as a test reagent, the optimum chain length may be selected according to the characteristics of the test reagent. However, when the particle size is too small, the sensitivity is lowered, and when the particle diameter is too large, clogging easily occurs. Therefore, the particle size is preferably 200 nm to 600 nm, and more preferably 300 nm to 500 nm.

Further, the chain length of the graft chain is one of the important factors that determine color of the particles. When the chain length is long, the agglutinating fluorescence tends to be strong, and when the chain length is short, the agglutinating fluorescence tends to be weak.

As described above, the chain length of the graft chain is preferably in the range of 10 nm to 240 nm.

A surface density of the graft chains in the polymer particles is preferably 0.05 to 0.20 chains/nm². When the surface density is higher than 0.20 chains/nm², there is concern about poor flowability on the membrane when used as an immunochromatographic reagent. When the surface density is lower than 0.05 chains/nm², there is concern about insufficient sensitivity.

A diagnostic immunochromatographic reagent using the particles of the present invention as a carrier for detection is also one of the present invention. Items of the diagnostic immunochromatographic reagent may include, for example, influenza virus, RS virus, adenovirus, rotavirus, norovirus and the like. Antibody-sensitized particles may be prepared by binding antibodies against the item to the immunochromatographic composite particles of the present invention, and may be used as the immunochromatographic reagents.

Further, when the diagnostic immunochromatographic reagent of the present invention is used, there is no need for a conventional step of including a conjugate pad, for example, in a diagnostic test strip using the principle of immunochromatography. In other words, an analyte is dropped on a membrane, and then the analyte binds to and aggregates with an antibody (or antigen) against an antigen (or antibody) as the analyte, which is immobilized as an immune reaction site on the membrane, thereby determining the presence of the analyte in a sample. This immunochromatographic method is also one of the present invention. Further, the particles of the present invention may also be used for flow through type immunoassay.

### [Use of Particles]

The fluorescent particles for a diagnostic reagent of the present invention may be appropriately used in various methods using biological reactions, such as an enzyme immunoassay method, a fluorescence immunoassay method, a latex agglutination method, an immunochromatography method, etc., these methods using an antigen-antibody reaction by binding to a surface antigen (or antibody).

The present invention provides an immunoassay reagent using the above-mentioned fluorescent particles for a diagnostic reagent.

In addition to the method of using the aggregation-induced emission fluorescent material to which the analyte binding site has been imparted in advance, the analyte binding site may be imparted after the aggregation-induced emission fluorescent material-containing particles are prepared. The method of imparting the analyte binding site to the surface of the particles is not particularly limited, and a conventionally known method may be used, for example, a binding method by physical adsorption such as immersion of fluorescent particles for diagnostic agents in a buffer solution containing an antigen (or antibody) and incubation for a predetermined time at a predetermined temperature, or a binding method by chemical adsorption. Among them, the chemical adsorption is more preferred, in which a carboxyl group of colored latex particles and an amino group in an antibody are crosslinked and bonded.

According to the present invention, it is possible to prepare fluorescent particles for diagnostic agents which exhibit sufficiently strong fluorescence, and when the fluorescent particles for diagnostic agents are used as a reagent for immunoassay, visual judgment may be remarkably improved and detection sensitivity may be improved. Further, since the degree of particle dispersion is low, lot reproducibility during preparation of the reagent is improved.

Specific uses of the fluorescent particles for diagnostic agents and terminology will be described below.

### (Test Strip)

A test strip of FIG. 2(A) includes a plastic adhesive sheet a; an insoluble membrane carrier b disposed on the plastic adhesive sheet b, the insoluble membrane carrier b having at least one detection portion c on which binding partners for an analyte are immobilized; and an absorption pad g which is disposed at one end of the insoluble membrane carrier b. Here, a plurality of the detection portions may be provided to test other items.

According to the test strip of FIG. 2(A), an analyte may be suddenly spread on the insoluble membrane carrier b, as shown in FIG. 2(B). Further, the above-described highly sensitive aggregation-induced emission fluorescent material-containing particles in the detection portion may be used to enable very easy visual confirmation in an inspection step, as shown in FIG. 2(C).

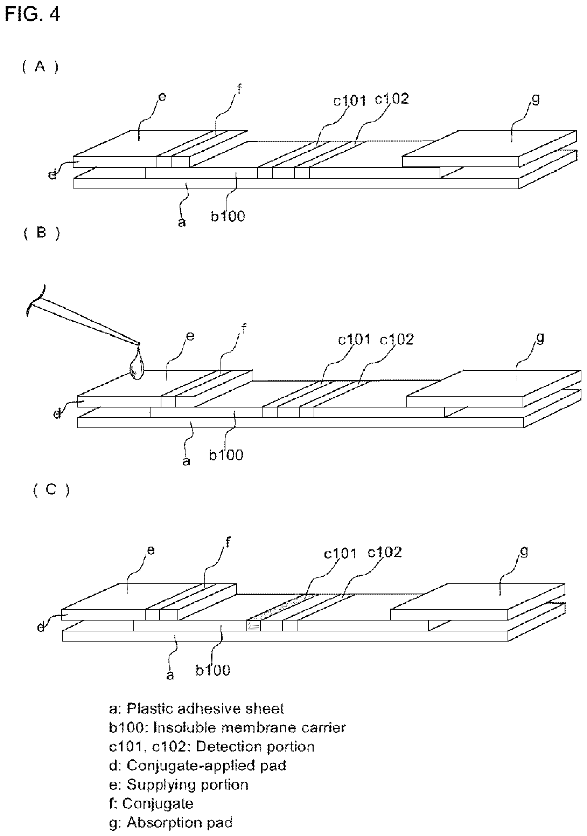

Meanwhile, a conventional immunochromatographic test strip shown in FIG. 4(A) requires a conjugate-applied pad d having conjugates f. As shown in FIG. 4(B), a step of sensitizing an analyte to the conjugate is required. There is also a problem of the visual observation in the inspection step, as shown in FIG. 4(C).

As described above, according to the test strip according to the embodiment of FIG. 2(A), since the step of sensitization to the conjugates is not required, the examination time may be shortened. In addition, it is possible to simplify the test strip and to reduce the cost, in terms of not requiring the conjugate-applied pad d. Further, use of the above-described highly sensitive aggregation-induced emission fluorescent material-containing particles enables much easier visual confirmation than the conventional test strip.

### (Aggregation-induced emission fluorescent material-Containing Inspection Device)

FIG. 3(A) is a perspective view of an inspection device 8 containing the aggregation-induced emission fluorescent material, FIG. 3(B) is a cross-sectional view thereof, and FIG. 3(C) is a conceptual diagram showing a usage state. As shown in FIG. 3(B), the aggregation-induced emission fluorescent material-containing inspection device 8 includes a sample container 10 as an insoluble carrier; and detection portions 11 and 12 placed in a line shape at the bottom of the sample container 10, the detection portions provided with aggregation-induced emission fluorescent materials. A diluent is contained in the sample container 10. A film-like cover such as polyethylene or polypropylene which covers the main surface of the sample container 10 may be provided such that it is removable during use. By providing a plurality of detection portions, a plurality of items may be tested. As shown in a partially enlarged view of FIG. 3(C), when a sample (possibly) containing an analyte is introduced into the sample container, the analyte 5 binds to binding partners 31, 32 of first and second graft chains 21, 22 of the aggregation-induced emission fluorescent material in the detection portions 11,12, whereby agglutinating fluorescence is generated and the presence of the analyte may be detected.

### EXAMPLES

Hereinafter, the present invention will be described in more detail with reference to Examples. However, the present invention is not limited to the constitution of the following Examples.

### [Preparation of aggregation-induced emission fluorescent material-Containing particles]

100 g of deionized water, 3.6 g (34 mmol) of styrene (manufactured by Kanto Chemical Co., Inc.), and 0.136 g (0.5 mmol) of a polymerization initiator V-50 (manufactured by Wako Pure Chemical Corp.) were added to a 200 mL three-necked flask equipped with a stirring blade, a reflux condenser, and a nitrogen inlet tube, and the flask was purged with nitrogen under stirring at 100 rpm, and polymerization was initiated at 60°C. After 4 hours from polymerization initiation, 0.375 g (1.7 mmol) of 2-chloropropionyloxyethyl methacrylate was added and polymerization was carried out for a total of 10 hours.

The obtained white solution was filtered through a mesh filter and purified by centrifugation (14, 500 rpm, 15 minutes, purification frequency of four times or more) to obtain target organic polymer microparticles (referred to as core particles 1a).

### [Addition (Preparation) of Graft Chain]

The core particle 1a (1.0 wt%, 30 mL) dispersed in water, 0.517 g (6.0 mmol) of MAA, copper (I) chloride/tris[2-(dimethylamino)ethyl]amine (150 µmol) as a metal complex, and 21.1 mg (120 µmol) of ascorbic acid as a reducing agent were added to a 100 mL two-necked flask, and the flask was purged with nitrogen under stirring by a stirrer, and polymerization was initiated at 30°C for 2 hours.

The obtained white solution was purified by centrifugation (14,500 rpm, 15 minutes, purification frequency of three times or more) to provide organic graft chains on the surface of the microparticles (referred to as first microparticles).

### [Addition of Aggregation-induced emission fluorescent material]

1.0 g of the first microparticles were dispersed in ethylene glycol, 0.578 g (1.46 mmoL) of tetrakis (4-hydroxyphenyl) ethylene, and 0.28 g (1.46 mmoL) of 1-ethyl-3-(3-dimethylaminopropyl) carbodiimide hydrochloride were added, and reaction was allowed at room temperature for 6 hours. The obtained dispersion was repeatedly purified by centrifugation using water (referred to as second microparticles).

### [Addition of Binding Partner]

A water dispersion (0.5 wt%, 10 mL) of the second microparticles and 2-aminoethanethiol (1 µmol) were added to a 20 mL sample bottle, and allowed to react under stirring at room temperature for 24 hours.

The obtained solution was purified by centrifugation (14,500 rpm, 20 minutes, purification frequency of four times or more) to obtain agglutinating-fluorescent-material-containing particles.

### [Application Example]

### <Preparation of Reagent for Measuring Influenza Virus>

### 1. Preparation of Composite Particle-Labeled Anti-Influenza A Virus Monoclonal Antibody

2 mL of a solution containing the above-mentioned aggregation-induced emission fluorescent material-containing particles was centrifuged at 12, 000 rpm for 5 minutes to precipitate, the supernatant was removed, and the precipitate was suspended in 20 mM MES buffer solution (pH 6.5) at a concentration of 2% by weight. To 500 µL of this particle suspension, 200 µL of 5 mg/mL influenza A monoclonal antibody (Clone # 622212), 160 µL of 15 mg/mL 1-ethyl-3-[3-(dimethylamino)propyl]carbodiimide (EDC), and 140 µL of 20 mM MES buffer solution (pH 6.5) were added and mixed with inversion at room temperature for 2 hours. Thereafter, the particles were precipitated by centrifugation at 12,000 rpm for 5 minutes, the supernatant was removed, and the precipitate was resuspended in 1 mL of a blocking buffer solution, and mixed with inversion at room temperature for 2 hours. The particles were precipitated by centrifugation at 12,000 rpm for 5 minutes again, the supernatant was removed, and then the precipitate was resuspended in 1 mL of the blocking buffer solution to obtain a reagent for measuring influenza virus.

The above blocking buffer solution has a composition of 2% bovine serum albumin (BSA) and a 50 mM tris buffer solution containing 10% sucrose (pH 8.5).

### 2. Fabrication of Insoluble Membrane Carrier

The reagent for measuring influenza virus thus prepared was diluted with a 10 mM phosphate buffer solution (pH 7.2) containing 2.5% sucrose at a concentration of 1.0 mg/mL to prepare a test line reagent. 1 µL/cm of test line reagent was applied using a dispenser (XYZ 3050, manufactured by Bio Dot, Inc.) on a nitrocellulose membrane having a width of 25 mm (manufactured by Sartorius Co., CN 140) at intervals of about 1 cm, and then dried in a dry oven at 70°C for 45 minutes to prepare an anti-influenza virus antibody-immobilized membrane.

### 3. Fabrication of Test Strip

The anti-influenza virus antibody-immobilized membrane (insoluble membrane carrier) (b) was attached to the center portion of the plastic adhesive sheet (a), and test lines (c1, c2) were disposed on the upstream side of spreading, and a control line (not shown) was disposed on the downstream side. An absorption pad (g) was placed and mounted on the downstream side while overlapping with both ends of the anti-influenza virus antibody-immobilized membrane. A structure obtained by superposing the respective components in this manner was cut into a width of 4 mm to fabricate a test strip shown in FIG. 2(A).

### 4. Preparation of Sample Extraction Solution and Sample for confirming Sensitivity

200 mM potassium chloride, 150 mM L-arginine, 0.25% BSA, 5% Starting Block (Thermo Fisher Scientific, No. 37542), and a 20 mM Tris buffer solution (pH 8.5) containing 0.5% Brij 35 (registered trade name: No. P1254-500 G of Sigma) were prepared as a sample extraction solution. Further, an inactivated influenza A virus solution was diluted with the sample extraction solution at 1.7 × 10⁶ TCID₅₀/mL to prepare a sample for confirming sensitivity.

### 6. Test Results

The test strip fabricated in the above described 4. was immersed in 135 µL of the sample for confirming sensitivity, and 10 minutes later, the presence or absence of color development of the type A test line and the control line was measured. Further, the color development measurement was carried out by visual evaluation of light emission when irradiated with ultraviolet rays (UV) having a wavelength of 365 nm.

### Example 1. Confirmation of effect of measuring method according to embodiment

### (Preparation Example: Preparation of PSA measuring reagent)

### 1. Second reagent solution (R2): Preparation of antibody-conjugated latex solution

(1) Anti-PSA monoclonal antibodies # 79 and # 91 and latex particles having an average particle diameter of 320 nm synthesized according to a standard method were each diluted with a 20 mM glycine buffer solution (pH 9) to prepare 0.7 mg/mL of each antibody solution and 1% (w/w) latex solution. Each of the antibody solutions was mixed with an equal amount of the latex solution and stirred for about 1 hour.
(2) An equal amount of a blocking solution (10% BSA) was added to each mixed solution of the above-described (1), and the mixed solutions were stirred for about 1 hour.
(3) Each of the mixed solutions of the above-described (2) was centrifuged to remove the supernatant, resuspended in a 5 mM MOPS buffer solution (pH 7.0) to adjust the absorbance at a wavelength of 600 nm to 1.5 Abs/mL. Then, equal amounts of both solutions were mixed to obtain a second reagent solution: antibody-conjugated latex solution (R2).

### 2. Preparation of first reagent solution (R1)

A 30 mM Tris-HCl buffer solution (pH 8.5) containing 1 M potassium chloride and 0.1% BSA was prepared and used as the first reagent solution.

### (Analyzer and Measurement Conditions)

Both scattered light intensity and absorbance were measured in a single measurement using an automatic analyzer described in JP-A-2013-64705. The measurement conditions of the scattered light intensity were established such that the wavelength of irradiated light was 700 nm and the scattering angle was 30 degrees. The measurement conditions of the absorbance were established such that the measurement was performed at two wavelengths consisting of the main wavelength of 570 nm and the sub-wavelength of 800 nm. To 8 µL of the sample containing PSA, 100 µL of R1 was added and stirred and then subjected to incubation for about 300 seconds at 37°C. Then, 100 µL of R2 was added and stirred and then was subjected to incubation for about 300 seconds at 37°C. Variations in the scattered light intensity and the absorbance were determined from differences in light quantity observed between selected two time points.

### (Calibration Curves and Sample Measurement)

The measured values of the scattered light intensity and the absorbance were respectively calibrated by spline using a PSA calibrator (manufactured by SEKISUI MEDICAL CO., LTD.) to plot respective calibration curves, which were used to determine PSA concentrations in the sample. The concentration range of the calibration curves was selected for each measurement depending on the dynamic range under each measurement condition.

### (Result 1: Sensitivity)

The variations in light quantity of the scattered light intensity and the absorbance were measured using the measuring method according to the present invention for measurement intervals of 270 seconds from about 30 seconds after the addition of R2, for which the highest sensitivity is supposed in determination of PSA according to embodiment. Samples containing PSA at different concentrations (0.4 ng/mL and 1 ng/mL, respectively) were serially analyzed ten times, and the reproducibility was confirmed for the measurement of the scattered light intensity and the measurement of the absorbance.

### (Result 2: Dynamic Range)

Dynamic ranges were compared under the following conditions with different measurement intervals: scattered light intensity (measurement intervals from about 30 seconds (first time point) to 270 seconds (second time point) after addition of R2, absorbance 1 (conditions 1 of the present invention: measurement intervals from about 30 seconds (third time point a) to about 90 seconds (fourth time point a) after addition of R2, absorbance 2 (conditions 2 of the present invention: measurement intervals from about 15 seconds (third time point b) to about 90 seconds (fourth time point b) after addition of R2, absorbance 3 (conventional conditions (Comparative Example): measurement intervals from about 30 seconds (third time point of Comparative Example) to 270 seconds (fourth time point of Comparative Example) after addition of R2.

### (Result 3: Observation of Influences of Prozone Effect)

Samples containing PSA at concentrations exceeding the range of from 100 ng/mL to 3000 ng/mL (collectively referred to as samples containing an ultra-high concentration of PSA) were analyzed under the measurement conditions for absorbances 1 and 2 according to the present invention, thereby observing the prozone effect. The prozone effect refers to a decrease in apparent measurements observed in particle enhanced agglutination immunoassay due to an excessive amount of antigen, and is a serious problem in clinical tests, because it may cause false-negative results and consequent misdiagnosis.

It is expected that the condition 2 showed a wider dynamic range with a more modest decrease in measurements due to the prozone effect. The results suggest that the practical upper limits of measurement for absorbances 1 and 2 are approximately 50 ng/mL and 100 ng/mL, respectively, and demonstrate that the measurement conditions for absorbance 2 allows a measurement range in a higher concentration range.

### (Result 4: Correlation)

PSA-positive samples containing known concentrations of PSA were analyzed by the measuring method according to embodiments to confirm correlation based on measurements of the scattered light intensity (●) and measurements of the absorbance 2 (Δ), respectively, in a low concentration range (10 ng/mL or less) and a high concentration range (10.1 ng/mL or more).

High correlation was observed and a particle enhanced agglutination immunoassay with high sensitivity and a wide dynamic range was achieved according to the present invention.

### INDUSTRIAL APPLICABILITY

According to the present invention, a step of sensitization to conjugates is not required in spite of an inspection method using antigen-antibody reaction, and therefore, the inspection time may be shortened, as compared with conventional immunochromatography. In addition, it is possible to simplify a test strip and to reduce the cost, in terms of not requiring a conjugate-applied pad. Further, use of highly sensitive aggregation-induced emission fluorescent material-containing particles enables much easier visual confirmation than the conventional test strip.

### REFERENCE NUMERALS

1 Aggregation-induced emission fluorescent material-containing particles
2 Graft chain
5 Analyte
21 First graft chain
22 Second graft chain
31 First binding partner
32 Second binding partner
8 Aggregation-induced emission fluorescent material-containing inspection device
10 Sample container
11,12 Detection portions

### ACCESSION NUMBER

### [REFERENCE TO DEPOSITED BIOLOGICAL MATERIALS]

(1) (Hybridoma #63279 producing #79 antibody)
   i) Name and address of depository institution at which the biological materials were deposited:
      International Patent Organism Depositary, National Institute of Advanced Industrial Science and Technology
      Tsukuba Central 6, 1-1-1 Higashi, Tsukuba, Ibaraki 305-8566, Japan
   ii) Date of biological material deposit in the depository institution in i):
      Feb. 19, 2010 (date of original deposit)
      (Thereafter, it was transferred from the original deposit (FERM P-21923) under the Budapest Treaty)
   iii) Accession number for the deposition assigned by the depository institution in i):
      FERM BP-11454
(2) (Hybridoma #63291 producing #91 antibody)
   i) Name and address of depository institution at which the biological materials were deposited:
      International Patent Organism Depositary, National
      Institute of Advanced Industrial Science and Technology Tsukuba Central 6, 1-1-1 Higashi, Tsukuba, Ibaraki 305-8566, Japan
   ii) Date of biological material deposit in the depository institution in i):
      Feb. 19, 2010 (date of original deposit)
      (Thereafter, it was transferred from the original deposit (FERM P-21924) under the Budapest Treaty)
   iii) Accession number for the deposition assigned by the depository institution in i):
      FERM BP-11455

## Claims

1. An analyte concentration measuring method comprising:
preparing a mixed solution by mixing a sample solution containing an analyte with a solution containing aggregation-induced emission fluorescent material-containing particles that have a binding partner which binds with the analyte and that agglutinate and fluoresce when the analyte binds to the binding partner;
measuring fluorescence intensity generated from the aggregation-induced emission fluorescent material-containing particles in the mixed solution;
comparing a fluorescence intensity calibration curve for an analyte concentration with the fluorescence intensity, and associating the fluorescence intensity with the analyte concentration in the mixed solution.

2. The analyte concentration measuring method of claim 1,
wherein the measuring the fluorescence intensity has at least one of steps as below:
measuring a variation in absorbance of the mixed solution from an absorbance difference between a first time point and a second time point and
measuring a scattered light intensity difference of the mixed solution from a scattered light intensity difference between a third time point and a fourth time point.

3. The analyte concentration measuring method of claim 1 or 2, wherein in the step of associating the analyte concentration, the fluorescence intensity is associated with the analyte concentration using the variation of the absorbance and/or the variation of the scattered light intensity and a calibration curve based on the variation of the scattered light intensity and/or a calibration curve based on the variation of the absorbance.

4. An aggregation-induced emission fluorescent material-containing particle comprising:
a core particle and
an aggregation-induced emission fluorescent material provided on the core particle,
wherein the aggregation-induced emission fluorescent material has a binding partner which binds with an analyte, and agglutinates and fluoresces when the analyte binds to the binding partner.

5. The aggregation-induced emission fluorescent material-containing particle of claim 4, wherein the aggregation-induced emission fluorescent material has an agglutinating fluorescent site localized on an insoluble carrier.

6. The aggregation-induced emission fluorescent material-containing particle of claim 5, wherein the aggregation-induced emission fluorescent material is provided as a graft chain on the surface of the insoluble carrier.

7. The aggregation-induced emission fluorescent material-containing particle of any one of claim 4 to 6, wherein the aggregation-induced emission fluorescent material further includes a hydrophilic group.

8. An inspection device comprising:
an insoluble carrier and
a detection portion which is provided on the insoluble carrier, the detection portion including an aggregation-induced emission fluorescent material which has a binding partner which binds to an analyte and agglutinates and fluoresces when the analyte binds to the binding partner.

9. The inspection device of claim 8, wherein the insoluble carrier is an insoluble membrane carrier.
